# EUROPEAN PATENT SPECIFICATION

(11) **EP 2 163 213 B1**
(45) Date of publication and mention of the grant of the patent: **15.08.2012**
(21) Application number: 09011745.8
(22) Date of filing: 15.09.2009
(51) Int. Cl.: A61B 17/29, A61B 17/00

(54) **Articulating surgical instrument using flexible fluid-filled tubing for transferring force**
Gelenkiges chirurgisches Instrument mit Verwendung von flüssigkeitsgefüllten Schläuchen zur Kraftübertragung
Instrument chirurgical articulé utilisant un tubage flexible rempli d'un fluide pour transferir une force

(30) Priority: 16.09.2008 US 211205
(43) Date of publication of application: 17.03.2010
(73) Proprietor: Tyco Healthcare Group, LP, North Haven CT 06473 (US)
(72) Inventor: Cunningham, James S., Boulder, CO 80304 (US); Nau, William H., Longmont, CO 80504 (US)
(74) Representative: HOFFMANN EITLE

(56) References cited:
- EP-A2- 1 785 101
- EP-A2- 1 842 500
- WO-A2-2004/028585
- DE-U1-202007 009 317
- US-A- 5 779 727

## Description

### BACKGROUND

### 1. Technical Field

The present disclosure relates to an apparatus for remotely activating jaw members on an articulating surgical instrument. In particular, the apparatus includes a hydraulic mechanism for appropriately transmitting force from a proximal end to a distal end of the instrument to cause a therapeutic effect on tissue clamped between the jaw members.

### 2. Background of Related Art

Typically in a laparoscopic, an endoscopic, or other minimally invasive surgical procedure, a small incision or puncture is made in a patient's body. A cannula is then inserted into a body cavity through the incision, which provides a passageway for inserting various surgical devices such as scissors, dissectors, retractors, or similar instruments. To facilitate operability through the cannula, instruments adapted for laparoscopic surgery typically include a relatively narrow shaft supporting an end effector at its distal end and a handle at its proximal end. Arranging the shaft of such an instrument through the cannula allows a surgeon to manipulate the proximal handle from outside the body to cause the distal end effector to carry out a surgical procedure at a remote internal surgical site. This type of laparoscopic procedure has proven beneficial over traditional open surgery due to reduced trauma, improved healing and other attendant advantages.

An articulating laparoscopic or endoscopic instrument may provide a surgeon with a range of operability suitable for a particular surgical procedure. The instrument may be configured such that the end effector may be aligned with an axis of the instrument to facilitate insertion through a cannula, and thereafter, the end effector may be caused to articulate, pivot or move off-axis as necessary to appropriately engage tissue. When the end effector of an articulating instrument comprises a pair of jaw members for grasping tissue, a force transmission mechanism such as a flexible control wire may be provided to open or close the jaws. For example, the control wire may extend through an outer shaft from the handle to the jaws such that the surgeon may create a tension in the control wire to cause the jaws to move closer to one another. The closure or clamping force generated in the jaws may be directly related to the tension in the control wire applied by the surgeon.

One type of laparoscopic or endoscopic instrument is intended to generate a significant closure force between jaw members to seal small diameter blood vessels, vascular bundles or any two layers of tissue with the application electrosurgical or RF energy. The two layers may be grasped and clamped together by the jaws of an electrosurgical forceps, and an appropriate amount of electrosurgical energy may be applied through the jaws. In this way, the two layers of tissue may be sealed together. The closure forces typically generated by this type of procedure may present difficulties when using a typical control wire to open and close the jaws of an articulating instrument.

For example, a surgeon's efforts to position the jaws may be frustrated by a tendency for a control wire under tension to realign the jaws with the axis of the instrument after the jaws have been articulated off-axis. Although this tendency may be observed in any type of articulating instrument, the tendency is particularly apparent when the closure forces and necessary tension in the control wire are relatively high, as is common with an electrosurgical sealing instrument. This tendency may be created by the direction of reaction forces through the outer shaft of the instrument.

EP 1 842 500 A2 discloses in figure 19 a surgical treatment tool in which a tube contains fluid for transmitting the opening and closing operation of the treatment section. DE 20 2007 009317 U1 discloses a hydraulic forceps with a pivoting handle and a stationary handle. The preamble of claim 1 is based on this document.

### SUMMARY

The present invention provides a surgical instrument as defined in claim 1. In a preferred embodiment, the present disclosure describes an endoscopic surgical instrument for sealing tissue including an elongated shaft defining a proximal shaft axis and a distal shaft axis.

The shaft axes may be misaligned to appropriately position and orient an end effector coupled to the distal end of the elongated shaft. The instrument is configured such that the shaft may resist a tendency to realign itself as the end effector moves from an open configuration for receiving tissue to a closed configuration for maintaining a closure pressure on the tissue ranging from about 3 kg/cm² to about 16 kg/cm². A handle is coupled to the proximal end of the elongated shaft, and is movable to selectively induce motion in the jaw members between the open configuration and the closed configuration. A fluid flow path extends between the handle and the end effector through the elongated shaft. The fluid flow path includes a master cylinder operatively associated with the handle to control a flow of a hydraulic fluid in the fluid flow path, and a follower cylinder responsive to the flow of the hydraulic fluid to move the jaw members between the open configuration and the closed configuration.

The elongated shaft includes at least one flexible portion therein such that the distal shaft axis and the proximal shaft axis may be selectively moved to a misaligned configuration. The elongated shaft may be flexible along a length thereof and sufficiently rigid to support the end effector in a position when the flexible shaft is in the misaligned configuration. Alternatively the elongated shaft may be constructed from a plurality of substantially rigid members that are pivotable relative to one another.

The hydraulic fluid may comprise a sterile 0.9% saline solution. The master cylinder may include a master piston having an effective area in contact with the hydraulic fluid that is substantially unequal from an effective area of a follower piston included in the follower cylinder. The effective area of the master piston may be less than the effective area of the follower piston. The jaw members of the instrument may be configured for bilateral movement.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1 is a perspective view of an articulating laparoscopic surgical instrument in accordance with the present disclosure;
FIG. 2A is a schematic view of the instrument of FIG. 1 depicting an end effector at a distal end of the instrument in a closed configuration; and
FIG. 2B is a schematic view of the distal end of FIG. 2A depicting the end effector in an open configuration.

### DETAILED DESCRIPTION

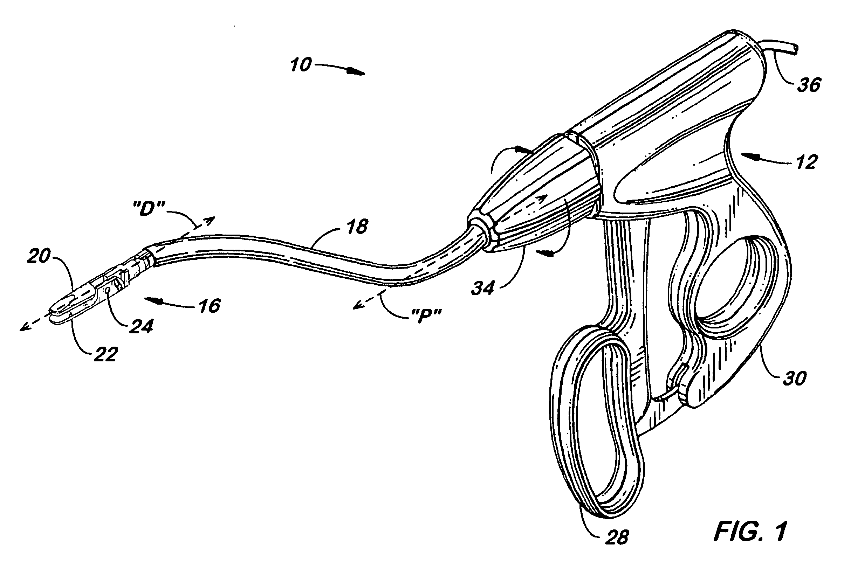

Referring initially to FIG. 1, an articulating endoscopic instrument is depicted generally as 10. The instrument 10 includes a handle portion 12 near a proximal end, an end effector 16 near a distal end and an elongated shaft 18 therebetween. Elongated shaft 18 defines a proximal shaft axis "P" substantially aligned with the handle portion 12 and a distal shaft axis "D" substantially aligned with the end effector 16.

The elongated shaft 18 is flexible such that the proximal shaft axis "P" and the distal shaft axis "D" may be selectively aligned or misaligned as shown. For example, the elongated shaft 18 may be bent in any direction, i.e., in both a horizontal and a vertical plane simultaneously, to assume a compound curve as shown. Other configurations are contemplated for elongated shaft 18, such as hinged or segmented wherein the shaft 18 is constructed from a plurality of substantially rigid members that are nested in series and/or pivotable relative to one another. A degree of rigidity may be maintained by the elongate shaft 18 such that the elongate shaft 18 supports itself to facilitate positioning of the end effector 16 at an anatomical position. Alternatively, a steering and locking mechanism (not shown) may be associated with the end effector 16 such that an operator may position the end effector 16 and lock the orientation of the elongated shaft 18 using a control surface (not shown) on the handle portion 12.

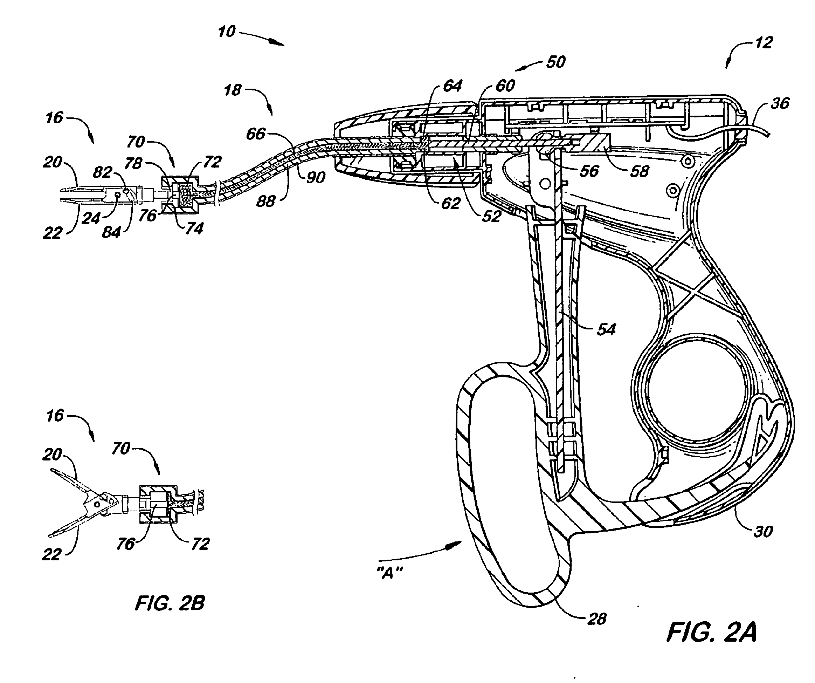

End effector 16 includes a pair of opposing jaw members 20 and 22. The jaw members 20, 22 are operable from the handle portion 12 to move between an open configuration (see FIG. 2B) to receive tissue, and a closed configuration to clamp the tissue and impart an appropriate clamping force thereto. When the end effector 16 is in the open configuration, a distal portion of each of the jaw members 20, 22 is spaced from the distal portion of the other of the jaw members 20, 22. In contrast, when the end effector 16 is in the closed configuration, the distal portions of the jaw members 20, 22 are closer together.

The motion exhibited by end effector 16 may be described as bilateral movement. Both jaw members 20 and 22 are pivotable about a pivot pin 24 such that each jaw member 20, 22 moves relative to distal shaft axis "D" as the end effector 16 is moved between the open and closed configurations. However, unilateral motion is also contemplated wherein an end effector (not shown) includes a stationary jaw that remains fixed relative to distal shaft axis "D" and a moveable jaw that moves relative to distal shaft axis "D" to move the end effector between the open and closed configurations. Further, non-pivotable motion is contemplated. For example, an end effector (not shown) may include jaw members configured for substantially parallel motion.

End effector 16 is configured for electrosurgical tissue sealing. At least one of the jaw members 20, 22 is connected to a source of electrical energy such that the electrical energy may be transmitted through tissue clamped between the jaw members. To form an effective tissue seal, a relatively high clamping force is typically generated to impart a closure pressure on the tissue in the range of from about 3 kg/cm² to about 16 kg/cm². As described in greater detail below, instrument 10 is configured to accommodate pressures in this range such that elongate shaft 18 exhibits no substantial tendency to inadvertently realign the proximal shaft axis "P" and the distal shaft axis "D." A more complete description of the tissue sealing generally may be found in U.S. Patent No. 7,367,976 to Lawes et al.

Handle portion 12 is manipulatable by the surgeon from outside a body cavity to control the movement of the end effector 16 positioned inside the body at a tissue site. For example, the surgeon may separate and approximate a pivoting handle 28 relative to a stationary handle 30 to respectively open and close jaw members 20, 22. Also, a surgeon may adjust an orientation of the jaw members by rotating knob 34. Handle portion 12 accommodates a cable 36 for delivery of an electrosurgical current to the instrument 10.

Referring now to FIG. 2A, instrument 10 is depicted schematically with end effector 16 in the closed configuration. Pivoting handle 28 is approximated to stationary handle 30, and distal portions jaw members 20, 22 are closer together relative to the open configuration depicted in FIG. 2B. A hydraulic mechanism 50 couples the end effector 16 to the pivoting handle 28 such that the jaw members 20, 22 may be remotely controlled from the handle portion 12.

Hydraulic mechanism 50 includes a master cylinder 52 housed in handle portion 12. Master cylinder 52 converts physical pressure applied by pivoting handle 28 into hydraulic pressure. To drive the master cylinder 52, pivoting handle 28 is coupled to a flexible arm 54, which engages a rounded head portion 56 of a connector 58. Connector 58 is, in turn, coupled to a master piston 60. Approximating pivoting handle 28 toward stationary handle 30 in the direction of arrow "A" pivots the flexible arm 54 against the rounded head portion 56. The rounded head portion 56 converts the pivotal motion of the pivoting handle 28 into longitudinal motion, which drives the connector 58 and master piston 60 in a distal direction. Driving the master piston 60 in a distal direction reduces the size of a proximal reservoir 62 defined between a cylinder wall 64 of master cylinder 52 and the master piston 60. Proximal reservoir 62 is filled with a hydraulic fluid, or other suitable fluid, such that a hydraulic pressure within reservoir 62 is increased as the size of the reservoir 62 is reduced.

Also filled with the hydraulic fluid is flexible tube 66, which is in fluid communication with proximal reservoir 62 through an aperture in cylinder wall 64. An increase in hydraulic pressure in proximal reservoir 62 results a flow of the hydraulic fluid through the tube 66 to a follower cylinder 70. Follower cylinder 70 is responsive to the increase in hydraulic pressure to move the jaw members 20, 22 between the open configuration and the closed configuration.

Follower cylinder 70 defines a distal reservoir 72 between a cylinder wall 74 and a follower piston 76. The distal reservoir 72 is in fluid communication with tube 66 through an aperture in the cylinder wall 74 such that the hydraulic fluid under pressure may flow into the distal reservoir 72 from the tube 66. This inflow of hydraulic fluid increases the size of distal reservoir 72 and drives the follower piston 76 in a distal direction.

The follower piston 76 is coupled to the end effector 12 such that the distal movement of the follower piston 76 tends to move the end effector 12 to the closed configuration. A bushing 78 supports the movement of follower piston 76 therethrough. A bridging member (not shown) may rigidly couple the bushing 78 to the pivot pin 24 such that a distance between the bushing 78 and pivot pin 24 remains constant. This allows a cam pin 82 driven by follower piston 76 through cam slot 84 to cause jaw member 22 to pivot about pivot pin 24. A similar arrangement on an opposite face of end effector 12 may cause jaw member 20 to also pivot about pivot pin 24.

In the process described above, approximation of pivoting handle 28 with stationary handle 30 results in the end effector 12 moving to the closed configuration as depicted in FIG. 2A. It follows that separation of pivoting handle 28 from stationary handle 30 results in the end effector 12 moving to the open configuration by the reverse process. The reverse process begins as flexible arm 54 drives the connector 58 in a proximal direction, which, in turn, draws the master piston 60 in the proximal direction. This increases the size of proximal reservoir 62 and draws in hydraulic fluid from tube 66. Flow of the hydraulic fluid through the tube draws fluid from the distal reservoir 72, decreasing the size of distal reservoir 72 and drawing follower piston in the proximal direction. This cams the jaw members 20, 22 to the open configuration as depicted in FIG. 2B.

Even with the relatively high clamping forces important for vessel sealing, the jaw members 20, 22 may be moved between the open and closed configurations when the distal shaft axis "D" is substantially misaligned with respect to the proximal shaft axis "P" without disruption of the placement of the end effector 16. The construction of the flexible shaft 18 may contribute, in part, to this functionality. Flexible shaft 18 includes a semi-rigid sleeve 88 covered by an insulation layer 90. The tube 66, filled with hydraulic fluid, is routed through the semi-rigid sleeve 88. The semi-rigid sleeve 88 provides sufficient structural strength to maintain a position of end effector 16, while tube 66 may have a relatively small inner diameter. For example, in one embodiment, tube 66 may be constructed with an inner diameter of 0.058 inches (1.5 mm) and maintain a hydraulic pressure of 600 p.s.i (40 kg/cm²).

The proximal and distal reservoirs 62, 72 may be configured with relatively large cross-sectional areas with respect to the inner diameter of tube 66. Since the diameter of the tube 66 is much less than the cross-sections of the proximal reservoir 62 and distal reservoir 72, the force required to be transmitted through the flexible shaft is virtually negligible when compared to the force that is experienced by the master piston 60 and the follower piston 76. The rigidity of semi-rigid sleeve 88 may be sufficient to absorb the forces transmitted through the flexible shaft 18 and thus resist the tendency for the flexible shaft to straighten when the jaw members are moved between the open and closed configurations.

To provide a mechanical advantage to the hydraulic mechanism 50, an effective area of the master piston 60 in contact with the hydraulic fluid is of unequal dimension with respect to an effective area of the follower piston 76. For example, as depicted in FIG. 2A, master piston 60 is smaller and travels through a longer stroke than follower piston 76. This allows a multiplication factor to be defined by the relationship between the effective areas of the pistons 60, 76. A smaller master piston 60 allows a smaller force to be applied at the handle portion 12 than is produced at the end effector 16. This may contribute to the ease of use of instrument 10. Alternatively, the effective area of the master piston 60 may be substantially equal to the effective area of the follower piston 76.

Various fluids may be effective as hydraulic fluids for use in the flow path defined by the hydraulic mechanism 50 between master cylinder 52 and the follower cylinder 70. As one example, the hydraulic fluid may be a sterile 0.9% saline solution so that leakage poses no danger of infection or harmful tissue reaction.

Although the foregoing disclosure has been described in some detail by way of illustration and example, for purposes of clarity or understanding, it will be obvious that certain changes and modifications may be practiced within the scope of the appended claims.

## Claims

1. A surgical instrument (10), comprising:
an elongated shaft (18) including distal and proximal ends, the distal end generally defining a distal shaft axis and the proximal end generally defining a proximal shaft axis;
an end effector (16) coupled to the distal end of the elongated shaft, the end effector including first and second opposable jaw members (20, 22) movable from an open configuration for receiving tissue to at least one closed configuration for maintaining a closure pressure on the tissue;
a handle (12) coupled to the proximal end of the elongated shaft, the handle selectively movable to induce motion in the jaw members between the open configuration and the closed configuration; and
a fluid flow path extending between the handle and the end effector through the elongated shaft, the fluid flow path including a master cylinder (52) operatively associated with the handle to control a flow of a hydraulic fluid in the fluid flow path and a follower cylinder (70) responsive to flow of the hydraulic fluid to move the jaw members between the open configuration and the closed configuration.
Wherein the handle includes a pivoting handle (28) and a stationary handle (30) **characterized in that** the pivoting handle is coupled to a flexible arm (54), the flexible arm engages a connector (58), and the connector is coupled to a master piston (60) of the master cylinder, wherein approximating the pivoting handle (28) toward the stationary handle (30) pivots the flexible arm (54) against the connector, which drives the connector distally or proximally, which, in tum, drives the master piston distally or proximally.

2. The instrument according to claim 1, wherein the elongated shaft includes at least one flexible portion therein such that the distal shaft axis and the proximal shaft axis may be selectively moved to a misaligned configuration.

3. The instrument according to claim 2, wherein the elongated shaft is flexible along a length thereof and sufficiently rigid to support the end effector in a position when the flexible shaft is in the misaligned configuration.

4. The instrument according to claim 1, 2 or 3, wherein the elongated shaft is constructed from a plurality of substantially rigid members that are pivotable relative to one another.

5. The instrument according to any one of the preceding claims, wherein the hydraulic fluid is a sterile 0.9% saline solution.

6. The instrument according to any one of the preceding claims, wherein the master piston has an effective area in contact with the hydraulic fluid and the follower cylinder includes a follower piston (76) having an effective area in contact with the hydraulic fluid, wherein the effective area of the master piston is substantially unequal to the effective area of the follower piston.

7. The instrument according to claim 6, wherein the effective area of the master piston is less than the effective area of the follower piston.

8. The instrument according to any one of the preceding claims, wherein the jaw members are configured for bilateral movement.

9. The instrument according to any one of the preceding claims, wherein the closure pressure ranges from about 3 kg/cm² to about 16 kg/cm².

## Patentansprüche

1. Chirurgisches Instrument (10), mit:
einem länglichen Schaft (18), der ein distales und ein proximales Ende aufweist, wobei das distale Ende im Allgemeinen eine distale Schaftachse definiert und das proximale Ende im Allgemeinen eine proximale Schaftachse definiert;
einem Endeffektor (16), der mit dem distalen Ende des länglichen Schafts verbunden ist, wobei der Endeffektor ein erstes und ein zweites entgegenstellbares Backenelement (20, 22) aufweist, das von einer offenen Anordnung zum Aufnehmen von Gewebe zu mindestens einer geschlossenen Anordnung zum Aufrechterhalten eines Schließdrucks auf das Gewebe bewegbar ist;
einem Griff (12), der mit dem proximalen Ende des länglichen Schafts verbunden ist, wobei der Griff gezielt bewegbar ist, um eine Bewegung der Backenelemente zwischen der offenen Anordnung und der geschlossenen Anordnung hervorzurufen; und
einem Fluiddurchflussweg, der sich zwischen dem Griff und dem Endeffektor durch den länglichen Schaft erstreckt, der einen Hauptzylinder (52) aufweist, der betriebsfähig mit dem Griff verbunden ist, um einen Fluss eines Hydraulikfluids in dem Fluiddurchflussweg zu steuern, und einen Folgezylinder (70), der auf einen Fluss des Hydraulikfluids anspricht, um die Backenelemente zwischen der offenen Anordnung und der geschlossenen Anordnung zu bewegen,
wobei der Griff einen schwenkbaren Griff (28) und einen feststehenden Griff (30) aufweist, **dadurch gekennzeichnet, dass** der schwenkbare Griff mit einem flexiblen Arm (54) verbunden ist, der einen Verbinder (58) in Eingriff bringt, und der Verbinder mit einem Hauptkolben (60) des Hauptzylinders verbunden ist, wobei ein Annähern des schwenkbaren Griffs (28) in Richtung des feststehenden Griffs (30) den flexiblen Arm (54) gegen den Verbinder schwenkt, was den Verbinder distal oder proximal antreibt, der wiederum den Hauptkolben distal oder proximal antreibt.

2. Instrument nach Anspruch 1, bei dem der längliche Schaft in sich mindestens einen flexiblen Abschnitt aufweist, sodass die distale Schaftachse und die proximale Schaftachse gezielt zu einer nicht fluchtenden Anordnung bewegt werden können.

3. Instrument nach Anspruch 2, bei dem der längliche Schaft entlang eines Abschnitts seiner Länge flexibel und ausreichend steif ist, um den Endeffektor in einer Position zu unterstützen, in der der flexible Schaft in der nicht fluchtenden Anordnung ist.

4. Instrument nach Anspruch 1, 2 oder 3, bei dem der längliche Schaft aus einer Vielzahl von im Wesentlichen steifen Elementen aufgebaut ist, die relativ zueinander schwenkbar sind.

5. Instrument nach einem der vorstehenden Ansprüche, bei dem das Hydraulikfluid eine sterile 0,9 % Salzlösung ist.

6. Instrument nach einem der vorstehenden Ansprüche, bei dem der Hauptkolben eine Wirkfläche aufweist, die mit dem Hydraulikfluid in Kontakt ist, und der Folgezylinder einen Folgekolben (76) aufweist, der eine Wirkfläche in Kontakt mit dem Hydraulikfluid aufweist, wobei die Wirkfläche des Hauptkolbens im Wesentlichen ungleich der Wirkfläche des Folgekolbens ist.

7. Instrument nach Anspruch 6, bei dem die Wirkfläche des Hauptkolbens kleiner als die Wirkfläche des Folgekolbens ist.

8. Instrument nach einem der vorstehenden Ansprüche, bei dem die Backenelemente für eine zweiseitige Bewegung eingerichtet sind.

9. Instrument nach einem der vorstehenden Ansprüche, bei dem der Schließdruck in einem Bereich von in etwa 3 kg/cm² bis in etwa 16 kg/cm² ist.

## Revendications

1. Instrument chirurgical (10), comprenant:
un arbre oblong (18) incluant des extrémités distale et proximale, l'extrémité distale définissant généralement un axe d'arbre distal, et l'extrémité proximale définissant généralement un axe d'arbre proximal;
un effecteur terminal (16) couplé à l'extrémité distale de l'arbre oblong, l'effecteur terminal incluant des premier et second éléments de mâchoire opposables (20, 22) déplaçables d'une configuration ouverte pour la réception du tissu à au moins une configuration fermée pour maintenir une pression de fermeture sur le tissu;
une poignée (12) couplée à l'extrémité proximale de l'arbre oblong, la poignée étant sélectivement mobile pour induire un mouvement dans les éléments de mâchoire entre la configuration ouverte et la configuration fermée; et
un chemin d'écoulement de fluide s'étendant entre la poignée et l'effecteur terminal à travers l'arbre oblong, le chemin d'écoulement de fluide incluant un cylindre maître (52) fonctionnellement associé à la poignée pour commander un écoulement d'un fluide hydraulique dans le chemin d'écoulement de fluide et un cylindre suiveur (70) réagissant à l'écoulement du fluide hydraulique pour déplacer les éléments de mâchoire entre la configuration ouverte et la configuration fermée,
où la poignée comprend une poignée pivotante (28) et une poignée stationnaire (30), **caractérisé en ce que** la poignée pivotante est couplée à un bras flexible (54), le bras flexible vient en prise avec un connecteur (58), et le connecteur est couplé à un piston maître (60) du cylindre maître, où le rapprochement de la poignée pivotante (28) vers la poignée stationnaire (30) fait pivoter le bras flexible (54) contre le connecteur, qui entraîne le connecteur distalement ou proximalement qui, à son tour, entraîne le piston maître distalement ou proximalement.

2. Instrument selon la revendication 1, où l'arbre oblong comprend au moins une portion flexible dans celui-ci de sorte que l'axe de l'arbre distal et l'axe de l'arbre proximal peuvent être sélectivement amenés à une configuration mal alignée.

3. Instrument selon la revendication 2, où l'arbre oblong est flexible sur une longueur de celui-ci et est suffisamment rigide pour supporter l'effecteur terminal dans une position où l'arbre flexible se trouve dans la configuration mal alignée.

4. Instrument selon la revendication 1, 2 ou 3, où l'arbre oblong est formé par une pluralité d'éléments sensiblement rigides qui peuvent pivoter les uns relativement aux autres.

5. Instrument selon l'une quelconque des revendications précédentes, où le fluide hydraulique est une solution saline stérile à 0,9%.

6. Instrument selon l'une quelconque des revendications précédentes, où le piston maître a une aire efficace en contact avec le fluide hydraulique, et le cylindre suiveur comprend un piston suiveur (76) ayant une aire efficace en contact avec le fluide hydraulique, où l'aire efficace du piston maître est sensiblement inégale à l'aire efficace du piston suiveur.

7. Instrument selon la revendication 6, où l'aire efficace du piston maître est inférieure à l'aire efficace du piston suiveur.

8. Instrument selon l'une quelconque des revendications précédentes, où les éléments de mâchoire sont configurés pour un mouvement bilatéral.

9. Instrument selon l'une quelconque des revendications précédentes, où la pression de fermeture est d'environ 3 kg/cm² à environ 16 kg/cm².
